# EUROPEAN PATENT SPECIFICATION

(11) **EP 1 676 599 B1**
(45) Date of publication and mention of the grant of the patent: **09.11.2011**
(21) Application number: 05257148.6
(22) Date of filing: 21.11.2005
(51) Int. Cl.: A61M 27/00

(54) **Ventriculostomy reservoir**
Ventrikulostomie-Reservoir
Réservoir de ventriculostomie

(30) Priority: 22.11.2004 US 995947
(43) Date of publication of application: 05.07.2006
(73) Proprietor: Codman & Shurtleff, Inc., Raynham, Massachusetts 02767 (US)
(72) Inventor: Kraus, Robert G., Attleboro, MA 02703 (US); Taylor, Robert, Coventry, RI 02816 (US); Buonanno, John, Bristol, RI 02809 (US)
(74) Representative: Mercer, Christopher Paul

(56) References cited:
- EP-A1- 0 402 571
- WO-A-83/01387
- WO-A-97/11726
- US-A- 5 387 188
- US-A1- 2004 193 094

## Description

The present invention relates to surgically implantable valves, and in particular to valves capable of resealable assembly and adapted for the drainage of cerebrospinal fluid from the ventricle of the brain.

Hydrocephalus is a condition afflicting patients who are unable to regulate cerebrospinal fluid flow through their body's own natural pathways. Produced by the ventricular system, cerebrospinal fluid (CSF) is normally absorbed by the body's venous system. In a patient suffering from hydrocephalus, the cerebrospinal fluid is not absorbed in this manner, but instead accumulates in the ventricles of the patient's brain. If left untreated, the increasing volume of fluid elevates the patient's intracranial pressure and can lead to serious medical conditions such as compression of the brain tissue and impaired blood flow to the brain.

The treatment of hydrocephalus has conventionally involved draining the excess fluid away from the ventricles and rerouting the cerebrospinal fluid to another area of the patient's body, such as the abdomen or vascular system. A drainage system, which usually includes a shunt valve, is often used to carry out the transfer of fluid. In order to install the shunt valve, typically a scalp incision is made and a small hole is drilled in the skull. A proximal, or ventricular, catheter is installed in the ventricular cavity of the patient's brain, while a distal, or drainage, catheter is installed in that portion of the patient's body where the excess fluid is to be reintroduced. Ventriculostomy reservoirs are often utilized in connection with such shunt valves to provide a convenient location for sampling accumulated cerebrospinal fluid as close to the brain ventricles as possible. Such ventriculostomy reservoirs may be placed over a burr hole through the skull to facilitate sampling of cerebrospinal fluid before the implantation of the fluid conduit.

Conventional ventriculostomy reservoirs typically include a metal base having a catheter connector, an integral, upwardly extending cylindrical wall portion, and a flange portion integrally formed with and overlying the wall portion. A cap made of a silicone elastomer material is typically provided to enclose the upper end of the base and define, with the base, an internal reservoir.

The cap and the base of such known ventriculostomy reservoirs are usually separated prior to implantation. The surgeon, after drilling a burr hole through the skull, attaches a catheter to the connector at the lower end of the base, positions the base, and assembles the cap.

EP 0402571 discusses a ventriculostomy reservoir which includes a rigid base and an elastomeric cap which is positioned over the base to enclose a reservoir well. A rigid cap insert is captured by the lower portion of the cap and is positioned for engagement with the base. The cap insert and the base are configured so that the cap may be snap-fit onto the base in a manner forming a fluid-tight seal therebetween.

One drawback of the conventional ventriculostomy reservoirs is the difficulty of manipulating the various components and assembling them into a fluid-tight and securely mated device within a patient. As will become apparent from the following description, the present invention satisfies these needs and provides other related advantages.

### SUMMARY OF THE INVENTION

The present invention generally provides a ventriculostomy reservoir device that is useful in treating hydrocephalus. The ventriculostomy reservoir device includes a base having an upper and lower opening, the upper opening defining an internal reservoir well, and a cap having an open bottom portion. The device further includes a flange element having a first portion disposed within a portion of the cap and a second portion extending distally beyond the open bottom portion of the cap and adapted for detachably mating with the internal reservoir well of the base. The detachable connection is facilitated by a snap fit element on the flange element that is adapted to cooperate with a complementary feature on the base to secure the flange element and the base together. The device also includes at least one sealing element disposed between the internal reservoir well and the flange element. The sealing element is adapted to provide a fluid tight seal between the base and the flange element when the base and the flange element are detachably mated.

Unlike prior art reservoirs with connector elements that perform both the securing and the sealing functions, the present invention includes separate snap fit and sealing elements. The snap fit element is particularly well adapted to provide a secure and detachable connection between the base and the flange, while the seal is particularly well suited to provide a fluid tight closure. For example, the seal can be formed from a more pliable material than the snap fit connection so that the seal can conform to the area between the base and the flange.

In one aspect of the invention, the snap fit element includes a protruding feature formed on the flange element that is matable with a complementary feature formed on the internal reservoir well. For example, the protruding feature can be formed on an outer surface of the flange element and a complementary recess can be formed on the base. The flange element and base are then mated by seating the flange element inside the internal reservoir well. In an alternative embodiment, the protruding feature can be formed on the base and the complementary feature can be formed on the flange element.

In another aspect of the invention, the snap fit element can include a split ring disposed in a recess on the flange element that is adapted to mate with a complementary feature formed on internal reservoir well. The split ring is sized and configured such that during assembly of the flange element and the base, the split ring is compressed. When assembly is complete, the split ring aligns with a recess and expands into position within the recess.

In yet another aspect of the invention, the snap fit element may take the form of multiple locking fingers, each of which is matable with a recess. For example, the base can include multiple locking fingers each having a protruding feature at a first end, and the flange element can include a complementary mating recess adapted to mate with the multiple locking fingers.

In a further aspect of the invention, the base can include an egress lumen having an upper portion and a lower portion. The snap fit element of the present invention can be formed between the egress lumen and the flange element. For example, the egress lumen can include a barb adapted to mate with a receiving area on the flange element.

The sealing element of the present invention can, in one aspect, be positioned within a sealing recess on the inner surface of the base and/or on the outer surface of the flange element. In another aspect, the device can include multiple seals in multiple sealing recesses.

### BRIEF DESCRIPTION OF THE DRAWINGS

The invention can be more fully understood from the following detailed description taken in conjunction with the accompanying drawings, in which:
FIG. 1 is a perspective view of one embodiment of the ventriculostomy reservoir device of the present invention;
FIG. 2A is a sectional view of the ventriculostomy reservoir device of FIG. 1;
FIG. 2B is a sectional view of the ventriculostomy reservoir device of FIG. 1 in a disassembled state;
FIG. 2C is a partially cut-away view of the ventriculostomy reservoir device of FIG. 1 in a disassembled state;
FIG. 3 is a sectional view of another embodiment of the ventriculostomy reservoir device of the present invention;
FIG. 4A is a sectional view of yet another embodiment of the ventriculostomy reservoir device of the present invention;
FIG. 4B is a partially cut-away view of the ventriculostomy reservoir device of FIG. 4A;
FIG. 5 is a sectional view of still another embodiment of the ventriculostomy reservoir device of the present invention;
FIG. 6A is a sectional view an alternative embodiment of the ventriculostomy reservoir device of the present invention;
FIG. 6B is a sectional view of the ventriculostomy reservoir device of FIG. 6A shown in a disassembled state;
FIG. 7A is a sectional view of an additional embodiment of the ventriculostomy reservoir device of the present invention; and
FIG. 7B is a perspective view of the ventriculostomy reservoir device of FIG.
7A.

### DETAILED DESCRIPTION OF THE INVENTION

In general, the present invention provides a ventriculostomy reservoir device having a reservoir area and a cap positionable over the reservoir area. A snap connection and a sealing element connect the reservoir area to the cap and thereby provide a detachable, fluid-tight connection. In one aspect, the snap connection is formed between non-elastomeric, substantially rigid materials such that the reservoir area and the cap are securely mated, while the seal is formed of a more pliable material to facilitate a fluid tight seal.

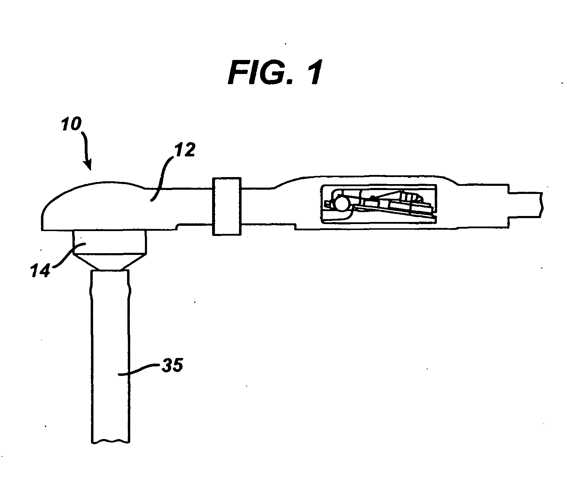

FIG. 1 illustrates an assembled ventriculostomy reservoir device 10, including a cap 12 and a base 14. The cap and the base are able to be mated to one another as described below and the assembled device encloses an internal reservoir well which can be sampled by inserting a sampling tool through cap 12.

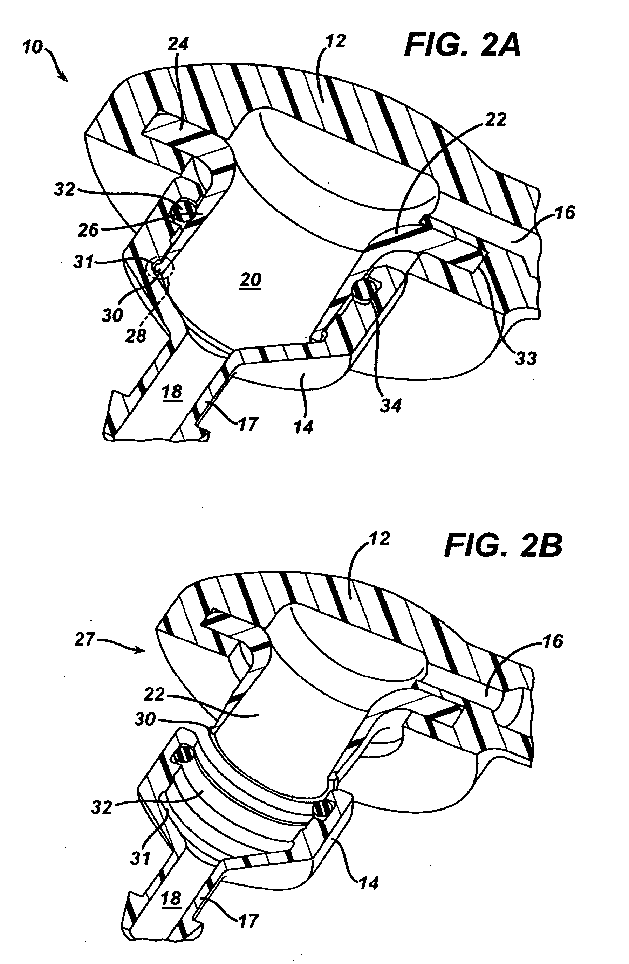

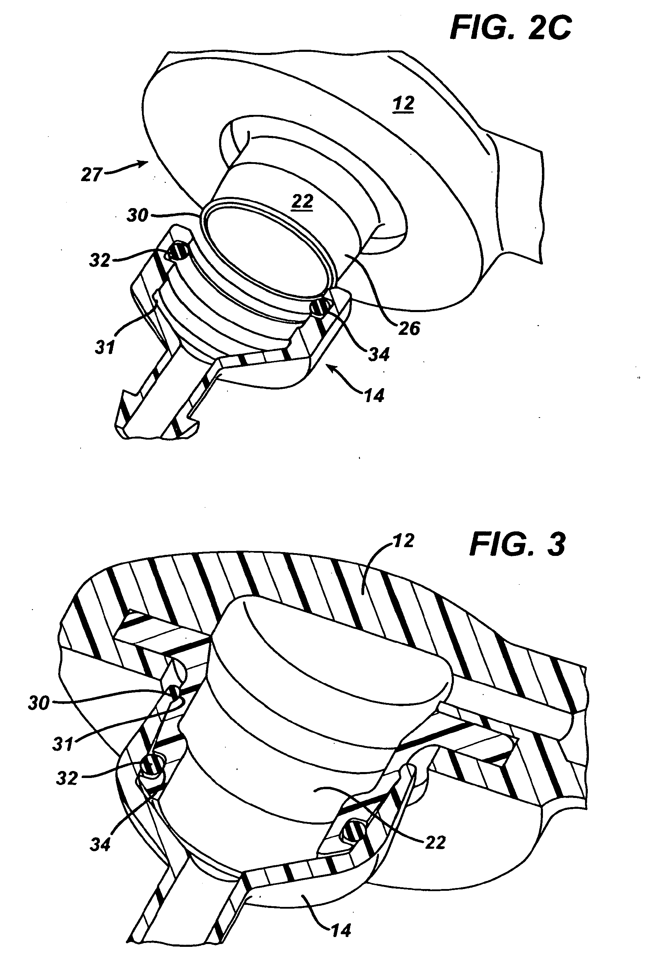

FIGS. 2A through 2C illustrate the construction of the device 10 pictured in FIG. 1. As shown, cap 12 includes a closed top and an open bottom portion. The ingress lumen 16 extends through the cap 12 into the internal reservoir well 20 via the open bottom portion. In another embodiment (not illustrated), the cap may include a closed bottom portion with the ingress lumen 16 feeding directly into internal reservoir well 20.

The base 14 defines an internal reservoir well 20 which is disposed below cap 12 when the ventriculostomy device is assembled. As shown, base 14 can have a funnel-like shape including an open top, side walls 13, and a narrow, lower tube 17. Sidewalls 13 can provide an a surface that is able to detachably mate with the flange element 22 and to enclose internal reservoir well 20. Lower tube 17 defines an egress lumen 18 that is adapted to drain fluid from the reservoir to another location or to another other implanted device. For example, lower tube 17 can mate with a catheter 35 (FIG. 1) and can include features, such as barb 36, to facilitate mating with the catheter.

Base 14 can be formed from a substantially rigid material so that it is able to maintain its shape after it is implanted and so that it can provide support to the portions of the device formed from a more pliable material, such as cap 12. The substantially rigid material properties of the base and the more pliable material properties of the cap would render them difficult to effectively and efficiently mate to one another during a surgical procedure. Accordingly, in one embodiment a substantially rigid flange element is used as a connecting element to join the cap 12 and base 14 together. In this embodiment, the pliable cap is fixedly mated with an upper portion 24 of rigid flange element 22, and the lower portion 26 of the flange element 22 is detachably mated with rigid base 14. The joinder of the cap and flange elements thus provides a cap/flange assembly 27 capable of securely and detachably mating to the base 14.

In an exemplary embodiment, illustrated in FIGS. 2A-2C, flange element 22 has an upper portion 24 that is in the form of an annular, substantially horizontally oriented ring. Integrally attached to upper portion 24 of flange element 22 is lower flange element portion 26, which is in the form of an annular ring that is oriented in a plane substantially orthogonal to upper portion 24. The upper portion 24 of flange element 22 is adapted to mate with the cap. One skilled in the art will appreciate that the flange can be mated to the cap 12 by a variety of techniques and that the engagement can be permanent or reversible. In an exemplary embodiment, however, upper portion 24 of flange element 22 is seated within a recess 33 formed in cap 12. The upper portion 24 can be maintained within recess 33 by an interference fit, or other techniques (e.g., use of adhesives, ultrasonic welding) may be used to secure the two components. In addition, upper portion 24 and/or the internal walls of recess 33 can have surface features that will enhance securement of the flange element to the cap.

As noted above, the flange element serves as an connecting element joining cap 12 and base 14. This can be accomplished by using lower portion 26 of flange element 22 to detachably mate with base 14. In one embodiment, a snap fit element can provide the secure, detachable connection between the lower portion 26 of the flange element 22 and the base 14. A variety of snap fit elements are contemplated, but they are generally characterized by the presence of opposed, complementary mating features on the flange element and the base. The invention encompasses the use of snap fit elements that are formed integrally with the base and flange element, as well as, snap fit elements that are separate from but matable to the flange element and the base.

In one example shown in FIGS. 2A-2C, a snap fit element 28includes a protruding feature 30 formed on an outer surface of the flange element 22 and a complementary recess 31 formed on an inner surface of base 14. In this embodiment, the flange/cap assembly 27 can be joined to the base by inserting the lower portion 26 of flange element 22 within the base, causing the lower flange portion 26 to slightly compress or deflect until protruding feature 30 reaches recess 31. At such point, the lower flange portion 26 will assume its original dimensions and feature 30 will be seated within recess 31. One skilled in the art will appreciate that the position of the protruding feature and the complementary recess can be reversed, as shown in FIG. 3, such that protruding feature 30 is positioned on base 14 and complementary recess 31 is positioned on lower flange portion 26. Further, it is understood that the feature 30 can be continuous, or it can be in the form of a plurality of discrete features.

In addition to snap fit element 28, a seal 32 can be used to enhance the mating of the flange element and the base, as well as to provide a fluid-tight connection between these components. Seal 32 is preferably positioned between flange element 22 and base 14, and in one embodiment, seal 32 is positioned between the inner surface of base 14 and the outer surface of flange element 22. The seal can be formed of a pliable material, such as an elastomer, such that the seal sufficiently conforms to the space between the flange and the base when the ventriculostomy reservoir is assembled.

The seal 32 can be seated in a seal recess 34 this can be formed in flange 22 or in base 14. The seal recess 34 helps to prevent migration of the seal when the flange/cap assembly 27 is joined to the base 14. In addition, since only a portion of seal 32 extends from the seal recess 34, the seal 32 creates minimal resistance to assembly when the flange element is inserted into the base.

In one embodiment, seal 32 is in the form of an o-ring positioned between base 14 and flange 22. For example, seal 32 shown in FIGS. 2A - 2C can be an o-ring having a circular cross section which will conform to the space between flange element 22 and base 14. One skilled in the art will appreciate that the cross section of the seal can have a variety of other shapes, such as, for example rectangular, triangular, or irregular. As shown, the overall shape of seal 32 can be a ring, and the diameter of the ring can be matched to that of base 14 and/or flange element 22. In one embodiment, the diameter of the seal can be slightly smaller than the diameter of the base so that the seal is stretched around base 14.

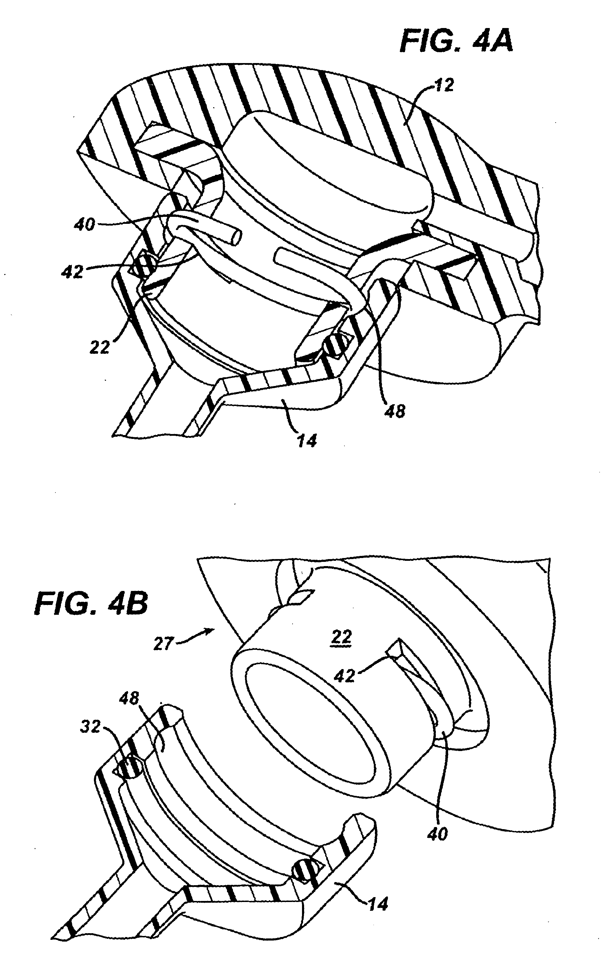

FIGS. 4A and 4B illustrate an alternative snap fit engagement that can be substituted for or be used with the protruding feature/complementary recess snap fit arrangement described above with respect to FIGS. 2A-3. As shown, the snap fit element is effected using a split ring 40 that joins the flange 22 to the base 14. The split ring 40 seats within a slot 42, and the split ring is of as size and shape such that a portion of the split ring extends beyond the perimeter of the slot 42, which as illustrated in FIGS. 4A and 4B, is formed in flange element 22. The portion of the split ring 40 that extends beyond the perimeter of slot 42 is adapted to seat within a complementary recess 48 formed in the base.

The split ring is generally circular and can have an opening 44 along one portion that allows the split ring to be compressed so that its diameter decreases. In addition, the split ring should be made from a resilient or shape-memory material such that one a compressive force is released, the split ring returns to its original configuration and dimensions. In use, the portion of the split ring that extends beyond the perimeter of the slot 42 is compressed as the flange element is slid within the base. Once the split ring is aligned with a recess 48 in the base, the compressive force on the split ring is removed and the split ring expands to occupy the recess 48. The split ring 40 thus acts to secure the flange/cap assembly to the base.

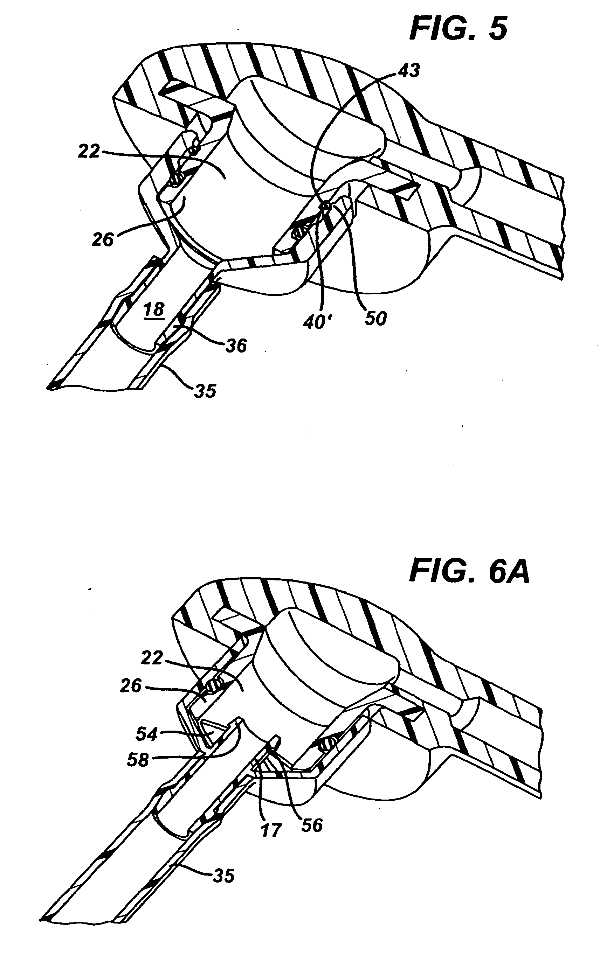

In an alternate embodiment, the split ring can snap around a raised portion of flange element 22 rather than snapping into a recess. For example, FIG. 5 illustrates ventriculostomy reservoir device 10 including a raised area 50 formed on the inner surface of base 14. The split ring 40' is seated within a groove 43 that is formed in the outer surface of the flange element 22. The relative dimensions of groove 43 and the split ring 40' are such that the split ring 40' remains seated in groove 43 during assembly of device 10, while still protruding to an extend sufficient to facilitate locking with base 12.

To mate base 14 to the cap/flange element assembly, the lower portion 26 of flange element 22 is slid within base 14. As the flange element is inserted, split ring 40' reaches the raised area 50 and is compressed. Once split ring 40' slides passed raised area 50, the split ring expands to provide a snap fit connection between flange element 22 and base 14. In yet another aspect, both raised area 50 and recess 48 can be used to hold the split ring within base 14.

In yet another aspect of a snap fit arrangement, a continuous ring (not illustrated) and a complementary feature can be used. For example, a ring similar to the split rings 40, 40', but lacking an opening 44, can be used to provide a snap fit connection. To assemble the cap/flange element with such a ring, the flange element is slid within the base. When the ring on the flange element reaches the complementary feature (e.g., a raised area), the base and/or the flange element slightly deforms to allow the ring to pass the raised area and snap fit into position behind the raised area. A continuous ring may also be used with a recess, with a similar slight deformation of the base required to snap the continuous ring of the flange element into the recess in the base.

One skilled in the art will appreciate that the location of the split or continuous ring can be varied. For example, a split or continuous ring could be positioned in base 14 instead of in flange element 22. In such an configuration, the location of the corresponding raised area or recess would be on the sidewall of flange element 22.

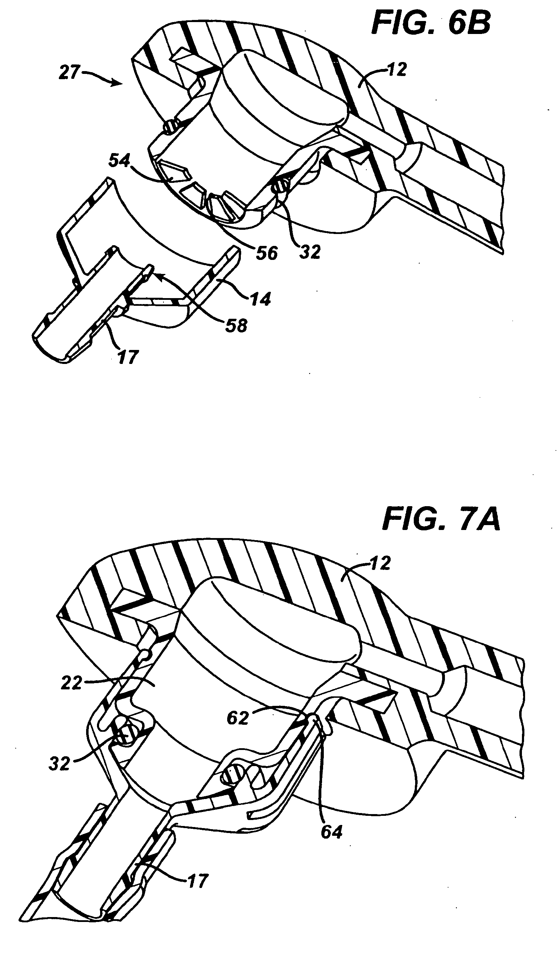

In another embodiment of the snap fit arrangement, a snap fit is formed between the bottom of internal reservoir well 20, defined by a bottom surface 54 of the flange element 22, and an upper portion of the lower tube 17. As shown in FIGS. 6A and 6B, the lower portion of the flange element extends across and encloses the bottom of the internal reservoir well with a bottom surface 54. The bottom surface 54 can be a continuous surface, or it can be formed of a plurality of discrete elements as shown in FIGS. 6A and 6B.

As further illustrated in FIGS. 6A and 6B, the base 14 includes a lower tube 17 that extends upwardly between the sidewalls 13 of base 14. An upper portion of the lower tube 17 includes surface features, such as barbs 58 to assist in the snap fit engagement. In addition, the bottom surface 54 includes an engagement tab 56 that is formed on an inner portion of the bottom surface of the flange element 22. The engagement tab 56 is complementary with the barbs 58 of the lower tube to facilitate snap fit engagement of the flange/cap assembly with the base.

FIGS. 6A and 6B also illustrate a seal 32 that can be positioned between the inner surface of the base 14 and the outer surface of the flange element 22 to provide a fluid tight seal. In addition, or alternatively, the seal can be positioned between bottom surface 54 of flange element 22 and base 14.

To assemble device 10, cap/flange assembly 27 is received between the sidewalls of base 14 until the barb on the lower tube 17 snap fits with the receiving area 54 of the flange element. In is understood that the lower tube 17 and/or lower surface 54 may deform to some extent during the assembly process.

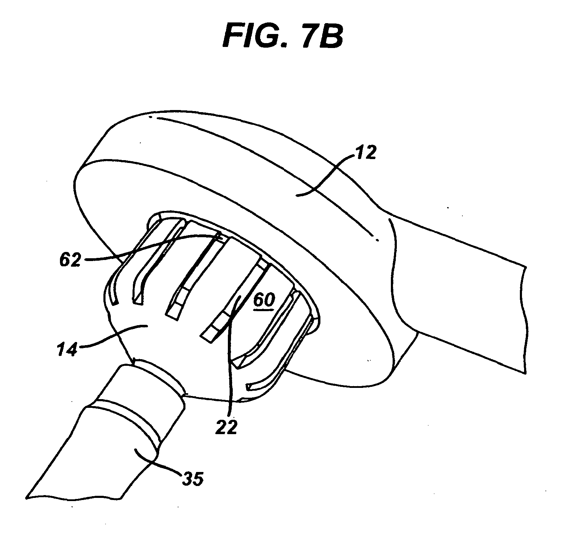

In yet another embodiment of the snap fit arrangement, snap fit fingers 60 formed on base 14 can snap into a recess 62 or behind a raised area (not shown) on flange element 22. In this embodiment illustrated in FIGS. 7A and 7B, snap fit fingers 60 extend from outer surface of base 14 and are adapted to mate with the outer surface of flange element 22. Snap fit fingers 60 can include a plurality of parallel, elongate members, each having a mating feature, such as protrusion 64, on the upper portion thereof. In addition, the outer surface of flange element 22 includes complementary recess 62 adapted to seat protrusions 64. When the device is assembled, the snap fit fingers encircle flange element 22 and snap fit into recess 62. This embodiment can also include seal 32 positioned between the inner surface of flange element 22 and the outer surface of the base 14 sidewalls 13 as shown in FIG. 7B.

One skilled in the art will appreciate that the exemplary snap fit arrangements disclosed above can secure the base and cap/flange assembly together such that the device will not accidentally dissassemble after implantation. For example, the cap flange/assembly can be mated to the base such that more than about 2.22 newtons (one half pound) of force is required to pull the assembled device apart, and in another aspect, more than about 4.45 newtons (1 pound) of force is required to pull the assembled device apart. In yet another aspect, the required force is in the range of about 4.45 to 44.48 newtons (1 to 10 pounds of force), and even more preferably 4.45 to 22.25 newtons (1 to 5 pounds of force).

As discussed above, the seal and the snap fit element can be formed from different materials. For example, the material used to form the seal is preferably a biocompatible, elastomeric material adapted to provide a fluid-tight seal between more rigid portions of the device. Exemplary materials that can be used to form the seal include, by way of non-limiting example, silicon rubber, fluoropolymers, and polyurethane. In one aspect, the materials used to form the seal can include materials having a hardness in the range of about 25 to 90 Shore A Durometer.

The substantially rigid material from which the base and flange element are formed can include various biocompatible materials that have sufficient strength to withstand implantation while retaining enough flexibility to allow a snap fit. Exemplary materials for forming the base and the flange include, nylon, polypropylene, fluoropolymers, ABS, polycarbonate, and stainless steel. While the same or different materials can be used to form the base, the flange element, and/or the snap fit element, the selected materials are preferably more rigid than seal 32.

One skilled in the art will appreciate further features and advantages of the invention based on the above-described embodiments. Accordingly, the invention is not to be limited by what has been particularly shown and described, except as indicated by the appended claims.

## Claims

1. A ventriculostomy reservoir device (10), comprising:
a base (14) having an upper and lower opening, the upper opening defining an internal reservoir well (20);
a cap (12) having an open bottom portion;
a flange element (22) having a first portion (24) disposed within a portion of the cap and a second portion (26) extending distally beyond the open bottom portion of the cap and adapted for detachably mating with the internal reservoir well of the base, the second portion of the flange element including a snap fit element (30) adapted to cooperate with a feature (31) on the internal reservoir well to secure the flange element and the base together; and
at least one sealing element (32) disposed between the internal reservoir well and the flange element,
the sealing element (32) being adapted to provide a fluid tight seal between the base (14) and the flange element (22) when the base and the flange element are detachably mated; and
wherein the sealing element (32) is disposed between an inner surface of the internal reservoir well and an outer surface of the flange element,
**characterised in that** the sealing element (32) and the snap fit element (30) are separate elements.

2. The device of claim 1, wherein the sealing element is formed of an elastomeric material that is more pliable than the base and the flange element.

3. The device of claim 1 or claim 2, wherein the snap fit element is substantially rigid.

4. The device of any one of claims 1 to 3, wherein the snap fit element on the second portion of the flange element is adapted to cooperate with a feature on an inner surface of the internal reservoir well.

5. The device of any one of claims 1 to 4, wherein the cap is formed from an elastomeric material and the flange element is formed from a non-elastomeric material.

6. The device of claim 5, wherein the cap is pliable and the flange element is substantially rigid.

7. The device of any one of claims 1 to 6, wherein the cap is constructed from silicone, polyurethane or a combination thereof.

8. The device of any one of claims 1 to 7, wherein the flange element is constructed from nylon, polypropylene, a fluoropolymer, ABS, polycarbonate, stainless steel or a combination thereof.

9. The device of any one of claims 1 to 8, including multiple sealing elements.

10. The device of any one of claims 1 to 9, wherein the sealing element is an o-ring.

11. The device of any one of claims 1 to 10, wherein the snap fit element includes a protruding feature formed on the flange element that is matable with a complementary feature formed on the internal reservoir well.

12. The device of claim 11, wherein the protruding feature is formed on an outer surface of the flange element.

13. The device of claim 12, wherein the protruding feature is integral with the flange element.

14. The device of any one of claims 1 to 10, wherein the snap fit element includes a recess (31) formed on the flange element that is matable with a complementary feature (30) formed on the on an inner surface of the internal reservoir well.

15. The device of any one of claims 1 to 14, wherein the snap fit element includes a split ring disposed in a recess on the flange element and adapted to mate with a complementary feature formed on internal reservoir well.

16. The device of any one of claims 1 to 15, wherein the feature on the internal reservoir well is a split ring disposed in a recess.

17. The device of any one of claims 1 to 16, wherein the first portion of the flange element is permanently joined to the cap.

18. The device of any one of claims 1 to 17, wherein at least about 4.45 newtons (1 pound) of force is required to separate the flange element and the base.

19. The device of any one of claims 1 to 18, wherein the force required to separate the detachably mated lower flange element from the base is in the range of 4.45 to 22.25 newtons (1 lb to 5 lbs).

20. The device of any one of claims 1 to 19, wherein the second portion of the flange element is positioned within an interior of the internal reservoir well.

21. The device of any one of claims 1 to 20, wherein the feature on the internal reservoir well includes multiple locking fingers (60) each of which is matable with the snap fit element.

22. The device of any one of claims 1 to 21, wherein the base includes multiple locking fingers (60) each having a protruding feature (64) at a first end, and wherein the snap fit element of the flange element includes a complementary mating recess (62) adapted to mate with the multiple locking fingers.

23. The device of any one of claims 1 to 22, wherein the feature on the internal reservoir well includes a first portion extending from an outer surface of the base, the first portion including at least one locking finger adapted to mate with the snap fit element disposed on an outer surface of the flange element.

24. The device of claim 23, wherein the sealing element is disposed between an outer surface of the internal reservoir well and an inner surface of the flange element.

25. The device of any one of claims 1 to 24, wherein the base includes an egress lumen (18) including an upper portion and a lower portion adapted to mate with a catheter (35).

26. The device of claim 25, wherein the snap fit element is adapted to cooperate with a feature on the upper portion of the egress lumen.

27. The device of claim 26, wherein the egress lumen includes a barb (36) adapted to mate with the second portion of the flange.

28. The device of any one of claims 1 to 27, wherein the cap includes an introduction channel (16).

## Patentansprüche

1. Ventrikulostomie-Behältervorrichtung (10), die Folgendes umfasst:
eine Basis (14), die eine obere und eine untere Öffnung hat, wobei die obere Öffnung eine innere Behälterwanne (20) definiert.
eine Kappe (12), die einen offenen Bodenabschnitt hat,
ein Flanschelement (22), das einen ersten Abschnitt (24), der innerhalb eines Abschnitts der Kappe angeordnet ist, und einen zweiten Abschnitt (26), der sich in Distalrichtung über den offenen Bodenabschnitt der Kappe hinaus erstreckt und dafür eingerichtet ist, lösbar mit der inneren Behälterwanne der Basis zusammenzupassen, hat, wobei der zweite Abschnitt des Flanschelements ein Schnappverbindungselement (30) einschließt, das dafür eingerichtet ist, mit einem Merkmal (31) an der inneren Behälterwanne zusammenzuwirken, um das Flanschelement und die Basis aneinander zu befestigen und
wenigstens ein Abdichtungselement (32), das zwischen der inneren Behälterwanne und dem Flanschelement angeordnet ist,
wobei das Abdichtungselement (32) dafür eingerichtet ist, eine fluiddichte Abdichtung zwischen der Basis (14) und dem Flanschelement (22) zu gewährleisten, wenn die Basis und das Flanschelement lösbar zusammengepasst sind, und
wobei das Abdichtungselement (32) zwischen einer Innenfläche der inneren Behälterwanne und einer Außenfläche des Flanschelements angeordnet ist,
**dadurch gekennzeichnet, dass** das Abdichtungselement (32) und das Schnappverbindungselement (30) gesonderte Elemente sind.

2. Vorrichtung nach Anspruch 1, wobei das Abdichtungselement aus einem elastomeren Werkstoff geformt ist, der biegsamer als die Basis und das Flanschelement ist.

3. Vorrichtung nach Anspruch 1 oder Anspruch 2, wobei das Schnappverbindungselement im Wesentlichen starr ist.

4. Vorrichtung nach einem der Ansprüche 1 bis 3, wobei das Schnappverbindungselement an dem zweiten Abschnitt des Flanschelements dafür eingerichtet ist, mit einem Merkmal an einer Innenfläche der inneren Behälterwanne zusammenzuwirken.

5. Vorrichtung nach einem der Ansprüche 1 bis 4, wobei die Kappe aus einem elastomeren Werkstoff geformt ist und das Flanschelement aus einem nicht-elastomeren Werkstoff geformt ist.

6. Vorrichtung nach Anspruch 5, wobei die Kappe biegsam ist und das Flanschelement im Wesentlichen starr ist.

7. Vorrichtung nach einem der Ansprüche 1 bis 6, wobei die Kappe aus Silikon, Polyurethan oder einer Kombination davon aufgebaut ist.

8. Vorrichtung nach einem der Ansprüche 1 bis 7, wobei das Flanschelement aus Nylon, Polypropylen, einem Fluorpolymer, ABS, Polycarbonat, rostfreiem Stahl oder einer Kombination davon aufgebaut ist.

9. Vorrichtung nach einem der Ansprüche 1 bis 8, die mehrere Abdichtungselemente einschließt.

10. Vorrichtung nach einem der Ansprüche 1 bis 9, wobei das Abdichtungselement ein O-Ring ist.

11. Vorrichtung nach einem der Ansprüche 1 bis 10, wobei das Schnappverbindungselement ein vorspringendes, an dem Flanschelement geformtes Merkmal einschließt, das mit einem komplementären, an der inneren Behälterwanne geformten Merkmal zusammengepasst werden kann.

12. Vorrichtung nach Anspruch 11, wobei das vorspringende Merkmal an einer Außenfläche des Flanschelements geformt ist.

13. Vorrichtung nach Anspruch 12, wobei das vorspringende Element integral mit dem Flanschelement ist.

14. Vorrichtung nach einem der Ansprüche 1 bis 10, wobei das Schnappverbindungselement eine an dem Flanschelement geformte Aussparung (31) einschließt, die mit einem an einem komplementären, an einer Innenfläche der inneren Behälterwanne geformten Merkmal (30) zusammengepasst werden kann.

15. Vorrichtung nach einem der Ansprüche 1 bis 14, wobei das Schnappverbindungselement einen Spaltring einschließt, der in einer Aussparung an dem Flanschelement angeordnet und dafür eingerichtet ist, mit einem komplementären, an der inneren Behälterwanne geformten Merkmal zusammenzupassen.

16. Vorrichtung nach einem der Ansprüche 1 bis 15, wobei das Merkmal an der inneren Behälterwanne ein in einer Aussparung angeordneter Spaltring ist.

17. Vorrichtung nach einem der Ansprüche 1 bis 16, wobei der erste Abschnitt des Flanschelements dauerhaft mit der Kappe verbunden ist.

18. Vorrichtung nach einem der Ansprüche 1 bis 17, wobei eine Kraft von wenigstens etwa 4,45 Newton (1 Pound) erforderlich ist, um das Flanschelement und die Basis zu trennen.

19. Vorrichtung nach einem der Ansprüche 1 bis 18, wobei die Kraft, die erforderlich ist, um das lösbar zusammengepasste untere Flanschelement von der Basis zu trennen, in dem Bereich von 4,45 bis 22,25 Newton (1 lb bis 5 lb) liegt.

20. Vorrichtung nach einem der Ansprüche 1 bis 19, wobei der zweite Abschnitt des Flanschelements innerhalb eines Inneren der inneren Behälterwanne angeordnet ist.

21. Vorrichtung nach einem der Ansprüche 1 bis 20, wobei das Merkmal der inneren Behälterwanne mehrere Einrastfinger (60) einschließt, von denen jeder mit dem Schnappverbindungselement zusammengepasst werden kann.

22. Vorrichtung nach einem der Ansprüche 1 bis 21, wobei die Basis mehrere Einrastfinger (60) einschließt, von denen jeder an einem ersten Ende ein vorspringendes Merkmal (64) hat, und wobei das Schnappverbindungselement des Flanschelements eine komplementäre Passaussparung (62) einschließt, die dafür eingerichtet ist, mit den mehreren Einrastfingern zusammenzupassen.

23. Vorrichtung nach einem der Ansprüche 1 bis 22, wobei das Merkmal der inneren Behälterwanne einen ersten Abschnitt einschließt, der sich von einer Außenfläche der Basis aus erstreckt, wobei der erste Abschnitt wenigstens einen Einrastfinger einschließt, der dafür eingerichtet ist, mit dem an einer Außenfläche des Flanschelements angeordneten Schnappverbindungselement zusammenzupassen.

24. Vorrichtung nach Anspruch 23, wobei das Abdichtungselement zwischen einer Außenfläche der inneren Behälterwanne und einer Innenfläche des Flanschelements angeordnet ist.

25. Vorrichtung nach einem der Ansprüche 1 bis 24, wobei die Basis ein Austrittslumen (18) einschließt, das einen oberen Abschnitt und einen unteren Abschnitt, der dafür eingerichtet ist, mit einem Katheter (35) zusammenzupassen, einschließt.

26. Vorrichtung nach Anspruch 25, wobei das Schnappverbindungselement dafür eingerichtet ist, mit einem Merkmal an dem oberen Abschnitt des Austrittslumens zusammenzuwirken.

27. Vorrichtung nach Anspruch 26, wobei das Austrittslumen einen Widerhaken (36) einschließt, der dafür eingerichtet ist, mit dem zweiten Abschnitt des Flanschs zusammenzuwirken.

28. Vorrichtung nach einem der Ansprüche 1 bis 27, wobei die Kappe einen Einführungskanal (16) einschließt.

## Revendications

1. Dispositif de réservoir de ventriculostomie (10), comprenant:
une base (14) présentant une ouverture supérieure et une ouverture inférieure, l'ouverture supérieure définissant une cavité de réservoir interne (20);
un bouchon (12) présentant une partie inférieure ouverte;
un élément de bride (22) présentant une première partie (24) qui est disposée à l'intérieur du bouchon, et une deuxième partie (26) qui s'étend de façon distale au-delà de la partie inférieure ouverte du bouchon et qui est adaptée pour se coupler de façon détachable avec la cavité de réservoir interne de la base, la deuxième partie de l'élément de bride comportant un élément d'emboîtement-pression (30) qui est adapté pour coopérer avec une caractéristique (31) sur la cavité de réservoir interne afin de fixer l'élément de bride et la base l'un à l'autre; et
au moins un élément d'étanchéité (32) qui est disposé entre la cavité de réservoir interne et l'élément de bride;
l'élément d'étanchéité (32) étant adapté pour établir une étanchéité au fluide entre la base (14) et l'élément de bride (22) lorsque la base et l'élément de bride sont couplés de façon détachable, et
dans lequel l'élément d'étanchéité (32) est disposé entre une surface intérieure de la cavité de réservoir interne et une surface extérieure de l'élément de bride,
**caractérisé en ce que** l'élément d'étanchéité (32) et l'élément d'emboîtement-pression (30) sont des éléments séparés.

2. Dispositif selon la revendication 1, dans lequel l'élément d'étanchéité est constitué d'une matière élastomère qui est plus pliable que la base et l'élément de bride.

3. Dispositif selon la revendication 1 ou la revendication 2, dans lequel l'élément d'emboîtement-pression est sensiblement rigide.

4. Dispositif selon l'une quelconque des revendications 1 à 3, dans lequel l'élément d'emboîtement-pression sur la deuxième partie de l'élément de bride est adapté pour coopérer avec une caractéristique sur une surface intérieure de la cavité de réservoir interne.

5. Dispositif selon l'une quelconque des revendications 1 à 4, dans lequel le bouchon est constitué d'une matière élastomère, et l'élément de bride est constitué d'une matière non élastomère.

6. Dispositif selon la revendication 5, dans lequel le bouchon est pliable, et l'élément de bride est sensiblement rigide.

7. Dispositif selon l'une quelconque des revendications 1 à 6, dans lequel le bouchon est constitué de silicone, de polyuréthane ou d'une combinaison de ceux-ci.

8. Dispositif selon l'une quelconque des revendications 1 à 7, dans lequel l'élément de bride est constitué de nylon, de polypropylène, d'un fluoropolymère, d'ABS (acrylonitrile-butadiène-styrène), d'un polycarbonate, d'acier inoxydable ou d'une combinaison de ceux-ci.

9. Dispositif selon l'une quelconque des revendications 1 à 8, comprenant de multiples éléments d'étanchéité.

10. Dispositif selon l'une quelconque des revendications 1 à 9, dans lequel l'élément d'étanchéité est un joint torique.

11. Dispositif selon l'une quelconque des revendications 1 à 10, dans lequel l'élément d'emboîtement-pression comporte une caractéristique saillante qui est formée sur l'élément de bride et qui peut se coupler avec une caractéristique complémentaire qui est formée sur la cavité de réservoir interne.

12. Dispositif selon la revendication 11, dans lequel la caractéristique saillante est formée sur une surface extérieure de l'élément de bride.

13. Dispositif selon la revendication 12, dans lequel la caractéristique saillante est intégrée à l'élément de bride.

14. Dispositif selon l'une quelconque des revendications 1 à 10, dans lequel l'élément d'emboîtement-pression présente un évidement (31) qui est formé sur l'élément de bride et qui peut se coupler avec une caractéristique complémentaire (30) qui est formée sur la surface intérieure de la cavité de réservoir interne.

15. Dispositif selon l'une quelconque des revendications 1 à 14, dans lequel l'élément d'emboîtement-pression comprend un anneau fendu qui est disposé dans un évidement sur l'élément de bride et qui est adapté pour se coupler avec une caractéristique complémentaire qui est formée sur la cavité de réservoir interne.

16. Dispositif selon l'une quelconque des revendications 1 à 15, dans lequel la caractéristique sur la cavité de réservoir interne est un anneau fendu qui est disposé dans un évidement.

17. Dispositif selon l'une quelconque des revendications 1 à 16, dans lequel la première partie de l'élément de bride est jointe au bouchon de façon permanente.

18. Dispositif selon l'une quelconque des revendications 1 à 17, dans lequel une force d'au moins environ 4,45 newtons (1 livre) est nécessaire pour séparer l'élément de bride et la base.

19. Dispositif selon l'une quelconque des revendications 1 à 18, dans lequel la force requise pour séparer l'élément de bride couplé de façon détachable de la base est comprise dans la gamme de 4,45 newtons à 22,25 newtons (1 livre à 5 livres).

20. Dispositif selon l'une quelconque des revendications 1 à 19, dans lequel la deuxième partie de l'élément de bride est positionnée à l'intérieur de la cavité de réservoir interne.

21. Dispositif selon l'une quelconque des revendications 1 à 20, dans lequel la caractéristique sur la cavité de réservoir interne comporte de multiples doigts de verrouillage (60) qui peuvent chacun être engagés avec l'élément d'emboîtement-pression.

22. Dispositif selon l'une quelconque des revendications 1 à 21, dans lequel la base comporte de multiples doigts de verrouillage (60) qui présentent chacun une caractéristique saillante (64) à une première extrémité, et dans lequel l'élément d'emboîtement-pression de l'élément de bride présente un évidement de couplage complémentaire (62) qui est adapté pour se coupler avec les multiples doigts de verrouillage.

23. Dispositif selon l'une quelconque des revendications 1 à 22, dans lequel la caractéristique sur la cavité de réservoir interne comprend une première partie qui s'étend à partir d'une surface extérieure de la base, la première partie comportant au moins un doigt de verrouillage adapté pour se coupler avec l'élément d'emboîtement-pression qui est disposé sur une surface extérieure de l'élément de bride.

24. Dispositif selon la revendication 23, dans lequel l'élément d'étanchéité est disposé entre une surface extérieure de la cavité de réservoir interne et une surface intérieure de l'élément de bride.

25. Dispositif selon l'une quelconque des revendications 1 à 24, dans lequel la base présente une lumière de sortie (18) comprenant une partie supérieure et une partie inférieure et qui est adaptée pour être raccordée à un cathéter (35).

26. Dispositif selon la revendication 25, dans lequel l'élément d'emboîtement-pression est adapté pour coopérer avec une caractéristique sur la partie supérieure de la lumière de sortie.

27. Dispositif selon la revendication 26, dans lequel la lumière de sortie comporte une barbe (36) qui est adaptée pour se coupler avec la deuxième partie de la bride.

28. Dispositif selon l'une quelconque des revendications 1 à 27, dans lequel le bouchon possède un canal d'introduction (16).
